# EUROPEAN PATENT SPECIFICATION

(11) **EP 1 609 501 B1**
(45) Date of publication and mention of the grant of the patent: **21.07.2010**
(21) Application number: 05253916.0
(22) Date of filing: 23.06.2005
(51) Int. Cl.: A61N 1/378, A61F 2/02

(54) **Medical implant having closed loop transcutaneous energy transfer (TET) power transfer regulation circuity**
Medizinisches Implantat mit einer Schaltung zur transkutanen Energieübertragung mit geschlossenem Kreislauf zur Übertragungsleistungssteuerung
Implant medical avec un circuit de transmission d'énergie par voie transcutanée (TET) à boucle fermée pour le reglage de la puissance de transmission

(30) Priority: 24.06.2004 US 876038
(43) Date of publication of application: 28.12.2005
(73) Proprietor: ETHICON ENDO-SURGERY, INC., Cincinnati, Ohio 45242 (US)
(72) Inventor: Hassler, William L. Jr., Cincinnati Ohio 45249 (US); Bloom, Gordon Edward, San Rafael California 94903 (US)
(74) Representative: Tunstall, Christopher Stephen

(56) References cited:
- US-A- 4 665 896
- US-A- 5 279 292
- US-A- 5 702 431
- US-A- 5 713 939
- US-A1- 2004 039 423
- US-B1- 6 442 434
- US-B1- 6 482 145

## Description

### Field of the Invention

The present invention relates, in general, to medically implantable devices that receive transcutaneous energy transfer (TET), and more particularly, such implant devices that regulate power transfer.

### Background of the Invention

In a TET system, a power supply is electrically connected to a primary coil that is external to a physical boundary, such as the skin of the human body. A secondary coil is provided on the other side of the boundary, such as internal to the body. With a subcutaneous device, both the primary and secondary coils are generally placed proximate to the outer and inner layers of the skin. Energy is transferred from the primary coil to the secondary coil in the form of an alternating magnetic field. The secondary coil converts the transferred energy in the AC magnetic field to electrical power for the implant device, which acts as a load on the secondary coil.

In a TET system, the primary and secondary coils are placed on separate sides of the boundary or skin. This separation typically results in variations in the relative distance and spatial orientation between the coils. Variations in the spacing can cause changes in the AC magnetic field strength reaching the secondary coil, in turn causing power fluctuations and surges in the implant device. Implant devices, such as those used in medical applications; usually rely upon a microcontroller to perform various functions. These microcontrollers require a consistent, reliable power source. Variations in the supplied power, such as sudden changes in voltage or current levels, may cause the device to perform erratically or fail to function at all. Accordingly, one issue associated with conventional TET systems is that the physical displacement of either the primary or secondary coils from an optimum coupling position may cause an unacceptable effect on the output power supplied to the implanted device. Additionally, the implant load on the secondary coil may vary as the device performs different functions. These load variations create different demands on the TET system, and lead to inconsistencies in the output power required to drive the load. Accordingly, it is desirable to have an accurate, reliable system for controlling the output power supplied to a load in a TET system. In particular, it is desirable to regulate the power induced in the secondary coil to provide an accurate, consistent load power despite variations in the load or displacement between the TET coils.

In U.S. Pat. No. 6,442,434, an energy transfer system is described wherein stable power is maintained in an implanted secondary circuit by having the secondary circuit generate a detectable indication that is sensed by the primary circuit. For instance, a voltage comparator in the secondary circuit senses that too much TET power is being received and shorts the secondary coil by closing a switch. The shorted secondary coil causes a current surge that is observable in the primary coil. The primary circuit is then adjusted so that these surges have a very small duty cycle, thus achieving voltage regulation since this condition indicates that the voltage in the secondary circuit is cycling close to a reference voltage used by the voltage comparator.

While apparently an effective approach to power regulation in a TET system, it is believed in some applications that this approach has drawbacks. For high impedance secondary coils, shorting the secondary circuit in this manner may create excessive heating, especially should the primary circuit continue to provide excessive power to the secondary circuit. Insofar as the '434 patent addresses continuous TET power of an artificial heart and other high power applications, such heating is a significant concern, warranting significant emphasis on modulating the power emitted by the primary circuit.

In U.S. Pat. No. 5,702,431, controlling current in a secondary circuit for battery charging is based upon switching capacitance into the secondary resonance circuit to change its efficiency. To that end, the AC resonance circuit is separated from the battery being charged by a rectifier. Current sensed passing through the battery is used to toggle two capacitors to vary the resonance characteristics of the secondary coil. The problem being addressed is providing a higher current during an initial stage of battery charging followed by a lower current to avoid damaging the battery due to overheating.

While these approaches to modifying power transfer characteristics of TET to a medical implant have applications in certain instances, it would be desirable to address the power requirements of a bi-directional infuser device suitable for hydraulically controlling an artificial sphincter. In particular, the power consumed to pump fluid is significant, as compared to what would be required for only powering control circuitry, for example. Moreover, powering the active pumping components need only occur intermittently. Since reducing the size of the medical implant is desirable, it is thus appropriate to eliminate or significantly reduce the amount of power stored in the infuser device, such as eliminating batteries.

Using TET to power the active pumping components, control circuitry and telemetry circuitry without the electrical isolation provided by a battery suggests that power regulation is desirable. In particular, most electronic components require a supply voltage that is relatively stable, even as the power demand changes. While having a primary circuit that is responsive to power transfer variability is helpful, such as alignment between primary and secondary coil, etc., it is still desirable that the implantable infuser device be relatively immune to changes in the power transferred. This becomes all the more desirable as rapidly changing power demands in the implanted medical device vary beyond the ability of the primary circuit to sense the change and respond.

US 4,665,896 discloses a power supply for a body implant and a method of use. US 6,482,145 B1 discloses a hydraulic anal incontinence treatment. US 5,279,292 discloses a charging system for implantable hearing aids and tinnitus maskers. US 5,713,939 discloses a data communication system for control of transcutatneous energy transmission to an implantable medical device. US 2004/0039423 A1 discloses the transmission of information from an implanted medical device.

Consequently, a significant need exists for an implantable medical device having secondary circuitry that optimizes power transfer characteristics from received transcutaneous energy transfer to power active components.

### Brief Summary of the Invention

The invention overcomes the above-noted and other deficiencies of the prior art by providing an implantable medical device and a transcutaneous energy transfer (TET) system as recited in the claims. The receiving circuitry performs voltage regulation sufficient to support active components, such as integrated circuitry, without resorting to batteries. Moreover, insofar as the receiving circuitry adjusts power transfer autonomously with regard to the primary circuitry, the implantable medical device is less susceptible to damage or inoperability due to variations in a power channel formed with the primary circuitry. In one aspect of the invention, an implantable medical device includes an active load that benefits from a stable electrical power supply with voltage remaining within a voltage range near a voltage reference even though the current demand may vary significantly. Thereby, a stable electrical power supply is provided to the active load.

These and other objects and advantages of the present invention shall be made apparent from the accompanying drawings and the description thereof.

### Brief Description of the Figures

The accompanying drawings, which are incorporated in and constitute a part of this specification, illustrate embodiments of the invention, and, together with the general description of the invention given above, and the detailed description of the embodiments given below, serve to explain the principles of the present invention.

FIG. 1 is a block diagram illustrating an exemplary energy transfer system in accordance with the present invention;

FIG. 2 is a block diagram illustrating a first embodiment for the power control system of the present invention;

FIG. 3 is a graphical representation of the output power from the secondary resonant circuit as modulated by the power control system;

FIG. 4 is a more detailed circuit diagram for the first embodiment of the power control system shown in FIG. 2;

FIG. 5A is a simplified circuit diagram depicting a first exemplary switching scheme for the power control system in a series resonant circuit;

FIG. 5B is a simplified circuit diagram depicting a second exemplary switching scheme for the power control system in a series resonant circuit;

FIG. 5C is a simplified circuit diagram depicting a third exemplary switching scheme for the power control system in a series resonant circuit;

FIG. 5D is a simplified circuit diagram depicting a fourth exemplary switching scheme for the power control system in a parallel resonant circuit;

FIG. 5E is a simplified circuit diagram depicting a fifth exemplary switching scheme for the power control system in a parallel resonant circuit;

FIG. 5F is a simplified circuit diagram depicting a sixth exemplary switching scheme for the power control system in a parallel resonant circuit;

FIG. 5G is a simplified circuit diagram depicting a seventh exemplary switching scheme for the power control system in a series resonant circuit;

FIG. 6 is a block diagram illustrating a second embodiment for the power control system of the present invention;

FIG. 7 is a schematic diagram depicting in more detail the power control system of FIG. 6;

FIG. 8 is a block diagram illustrating a third embodiment for the power control system of the present invention; and

FIG. 9 is a block diagram illustrating a fourth embodiment for the power control system of the present invention.

### Detailed Description of the Invention

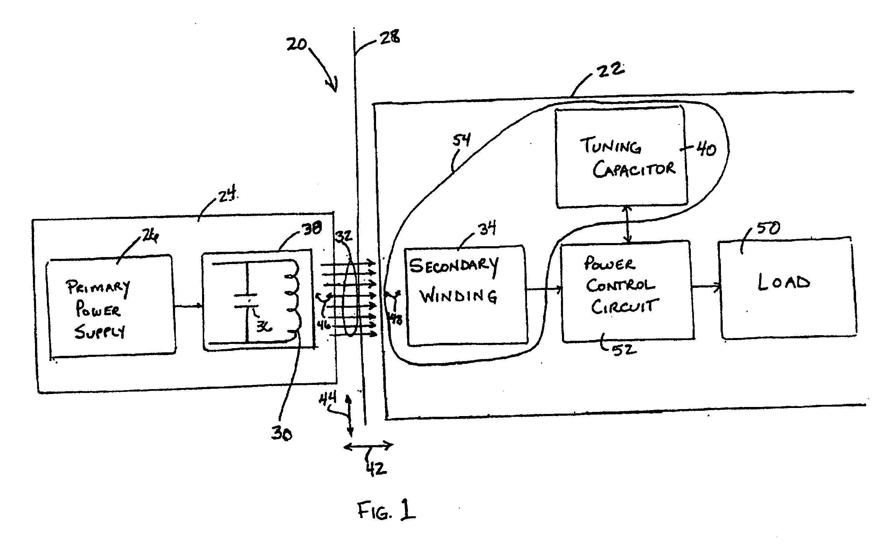

Referring now to the drawings in detail, wherein like numerals indicate the same elements throughout the views, FIG. 1 illustrates a transcutaneous energy transfer (TET) system 20 for an implant device 22 in accordance with the present invention. TET system 20 includes a primary circuit 24 comprising a power supply 26 located external to a physical boundary 28. Boundary 28 may be the skin of a human or animal body, such as in the case of a medical implant, or may be any other type of inanimate material or tissue depending upon the particular application of TET system 20. Primary circuit 24 also includes a primary coil 30 and one or more capacitors 36. Capacitor 36 is connected in parallel with primary coil 30 to form a primary resonant circuit 38. Primary resonant circuit 38 is electrically coupled to power supply 26 to resonate at the desired power signal frequency. An alternating magnetic field 32 is generated in primary coil 30 in response to input power provided by power supply 26.

A secondary coil 34 is provided in a spaced relationship from primary coil 30. Typically secondary coil 34 will be located on the opposite side of boundary 28 from primary coil 30. In the discussion herein, secondary coil 34 is located within implant device 22. Secondary coil 34 is electrically coupled to primary coil 30 via alternating magnetic field 32, symbolically illustrated in the figures as arrows emanating from primary coil 30 and propagating towards secondary coil 34. Secondary coil 34 is electrically connected in series with one or more tuning capacitors 40. Tuning capacitor 40 is selected to enable coil 34 and tuning capacitor 40 to resonate at the same frequency as primary resonant circuit 38. Accordingly, first and second coils 30, 34 and corresponding capacitors 36,40 form a pair of fixed power resonator circuits which transfer a maximum amount of energy between power supply 26 and implant 22 at the resonant frequency.

As shown in FIG. 1, primary coil 30 and secondary coil 34 are usually positioned relative to each other such that the secondary coil intercepts at least a portion of alternating magnetic field 32. While primary coil 30 and secondary coil 34 are magnetically coupled, the coils are typically not physically coupled. Accordingly, coils 30, 34 may be moved relative to each other, and the energy coupling between the coils may vary depending upon the relative displacement between the coils. The relative displacement between the coils 30, 34 may be in an axial direction as indicated by reference numeral 42. Similarly, the displacement between coils 30, 34 may be in a lateral direction, essentially orthogonal to the axial displacement, as indicated by reference numeral 44. The displacement between coils 30, 34 could also consist of a change in the angular orientation of one coil relative to the other coil, as indicated by reference numerals 46 and 48. Each of these various displacements between coils 30, 34 can cause a change in the amount of alternating magnetic field 32 reaching the secondary coil 34. The power induced in secondary coil 34 is inversely related to the displacement between coils 30, 34. The greater the displacement between coils 30, 34, the lower the amount of power induced in secondary coil 34. As primary coil 30 moves relative to secondary coil 34 (such as when primary circuit 24 is manipulated by a medical practitioner in the case of a medical implant) the power induced in secondary coil 34 can swing from very high to very low voltage and/or current levels.

Secondary coil 34 is electrically coupled to a load 50 and provides output power to the load from the received magnetic field 32. Depending upon the particular application, load 50 may represent one or more of a variety of devices that use the output power provided by secondary coil 34 to perform different operations. Load 50 may be associated with some resistance or impedance that, in some applications, may vary from time to time during normal operation of the load depending, in part, on the particular function being performed. Accordingly, the output power required by load 50 may also vary between different extremes during operation of implant 22.

In order to respond to these inherent power variations and provide a stable power supply to load 50, the present invention includes a power control circuit 52. Power control circuit 52 interfaces with secondary coil 34 and tuning capacitor 40 to control the power transfer from the primary coil 30. Power control circuit 52 measures the power signal from a secondary resonant circuit 54, formed by the combination of the secondary coil 34 and the tuning capacitor 40, and based upon the measured value, pulse width modulates the power signal to produce an output voltage at an acceptable level for implant load 50.

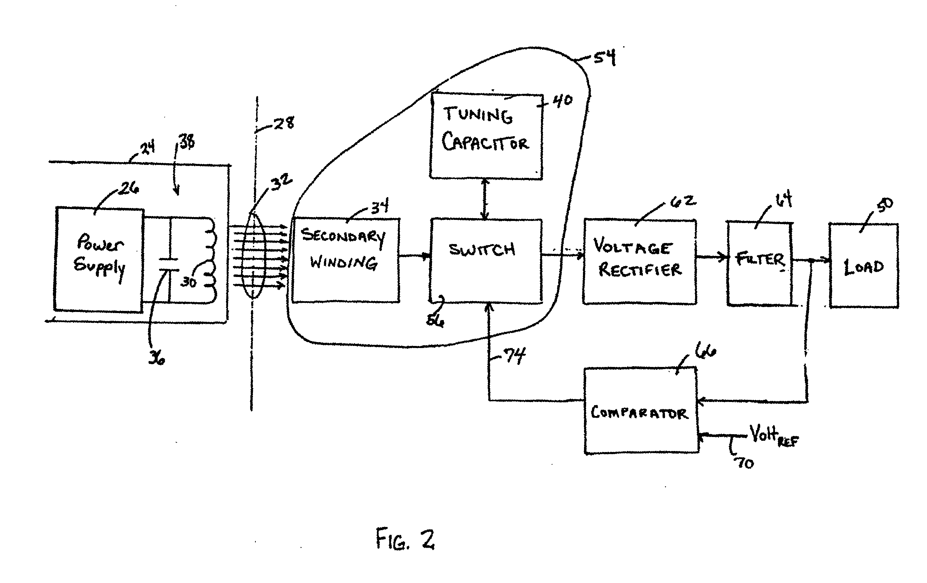

In a first embodiment shown in FIG. 2, power control circuit 52 comprises a switch 56 that internally modulates the power signal induced in secondary coil 34 to control the power output to load 50. Switch 56 modulates the power signal by selectively detuning secondary resonant circuit 54 when the voltage output to load 50 exceeds a predetermined threshold level. A suitable switch 56 may include a solid state switch such as a triac or silicon controlled rectifier (SCR). The secondary resonant circuit 54 is detuned by placing switch 56 in the resonant circuit, and selectively closing the switch 56 to short-circuit either tuning capacitor 40 or secondary coil 34. Short-circuiting either capacitor 40 or coil 34 causes secondary resonant circuit 54 to lose resonance, thereby preventing energy transfer through coil 34 to load 50. When the load voltage drops below the voltage threshold, switch 56 is opened to again transfer power to load 50. By repeatedly detuning and then retuning secondary resonant circuit 54, to stop and start energy transfer through secondary coil 34, power control circuit 52 modulates the output power from coil 34 into a series of power pulses.

It should be appreciated by those skilled in the art that selectively detuning to manage power transfer may be in response to sensed load current in addition to, or as an alternative to, sensed load voltage.

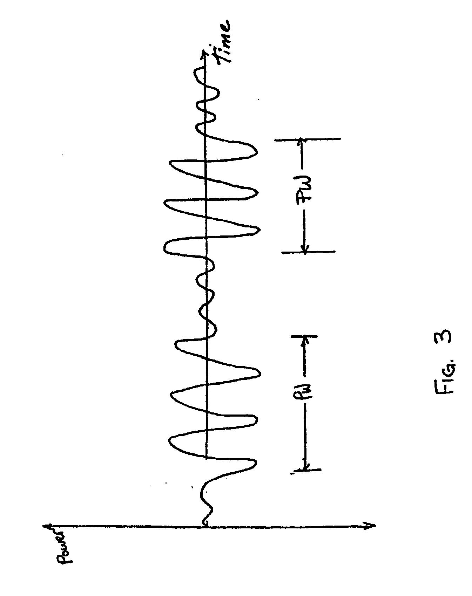

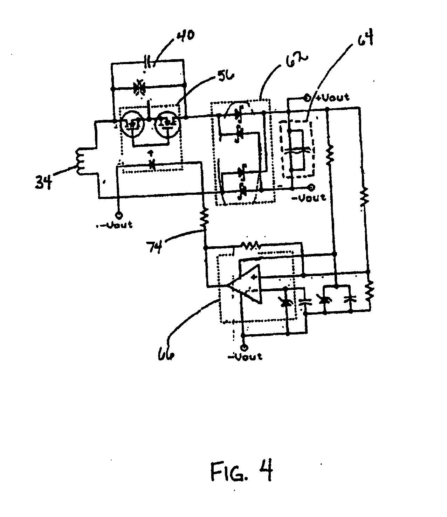

FIG. 3 depicts an exemplary series of power pulses corresponding to the selective tuning and detuning of resonant circuit 54. As the distance between primary and secondary coils 30, 34 varies, the width of the power pulses (indicated as PW in FIG. 3) will vary to adjust the output power to load 50. The smaller the relative displacement between the coils 30, 34, the shorter the power pulses necessary to generate the desired load power output. Conversely, the greater the displacement between primary and secondary coils 30, 34, the greater the period of time switch 56 is opened in order to transfer sufficient power to drive load 50. As the load power requirements vary, the pulse width PW will also vary. When load 50 requires an increased amount of power, such as to drive a motor or operate an element within implant 22, the pulse width PW or switch open time will increase to allow more power to be applied to the load. A full-wave rectifier 62 rectifies the pulse width modulated power signal. In addition, one or more filter capacitors 64, shown in FIG. 4, filter the power signal before it is applied to load 50.

To determine when the induced power signal exceeds the voltage threshold for load 50, power control circuit 52 includes a comparator 66 shown in FIG. 2. Comparator 66 compares the output voltage for load 50 with a predetermined threshold voltage level 70. The threshold voltage level 70 may be the maximum desired operating voltage for the implant load 50. Comparator 66 outputs a signal 74 that varies continuously in proportion with the difference between its inputs, namely the output voltage from filter capacitors 64 and the reference voltage (i.e., voltage threshold 70). Comparator output 74 is coupled to switch 56 to activate the switch 56 based upon the comparison between the output load voltage and the threshold voltage 70. When output signal 74 from comparator 66 reaches the activation point for switch 56, indicating an increase in the voltage level beyond the acceptable operating range, switch 56 is activated to short circuit the resonant circuit 54. Likewise, when the output voltage from capacitors 64 drops below an acceptable level for implant operation, such as when either the load demand, relative displacement between the coils 30, 34, or both increase, then output signal 74 of comparator 66 triggers switch 56 to open, thereby enabling power to again be induced and transferred through secondary coil 34.

FIG. 4 provides a more detailed, exemplary schematic diagram for the first embodiment of the present invention. As shown in FIG. 4 in the first embodiment, switch 56 is placed in parallel with tuning capacitor 40 in order to short-circuit the capacitor from resonant circuit 54 when the switch 56 is closed. Switch 56 is depicted as a solid-state relay that is flipped on or off when output signal 74 from comparator 66 reaches the set point, Also in this exemplary embodiment, voltage rectifier 62 is a full-wave bridge rectifier comprised of four Schottky diodes connected to rectify or demodulate the power signal from power circuit 52. Capacitors 64 filter the rectified power signal before application to load 50.

As mentioned above, FIG. 4 depicts switch 56 as a solid-state relay in parallel with capacitor 40 for pulse width modulating resonant circuit 54. In addition to this switching configuration, numerous other embodiments may also be utilized for selectively decoupling secondary coil 34 from primary coil 30 to regulate power transfer to the implant. Any available circuit topology may be employed in the present invention that would achieve the selective decoupling of the TET coils 30, 34 in response to the variations in transfer power.

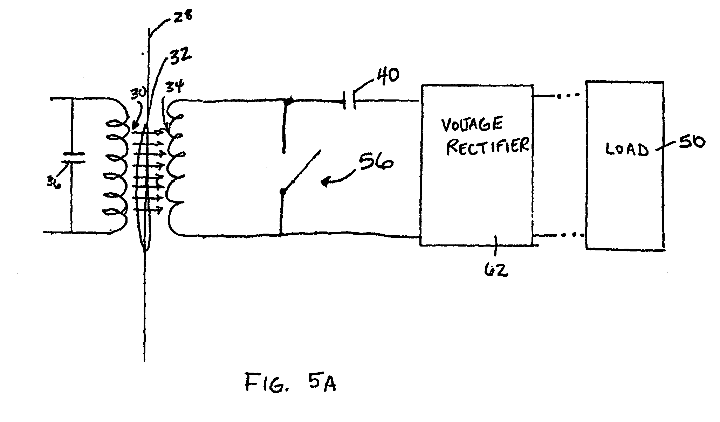

FIGS. 5A through 5G illustrate several exemplary circuit topologies that may be implemented to achieve power regulation in accordance with the invention. FIG. 5A illustrates one embodiment for selectively short-circuiting secondary coil 34 when the coil and tuning capacitor 40 form a series resonant circuit. Switch 56 is selectively turned on by comparator output signal 74, which is not shown in FIGS. 5A-5G, when the voltage induced in secondary coil 34 exceeds voltage threshold 70. When switch 56 is turned on, switch 56 forms a short circuit across secondary coil 34 to detune resonant circuit 54 and prevent energy transfer from the secondary coil. When switch 56 is turned off, the short circuit (or detuning) is removed and secondary circuit 54 returns to resonance.

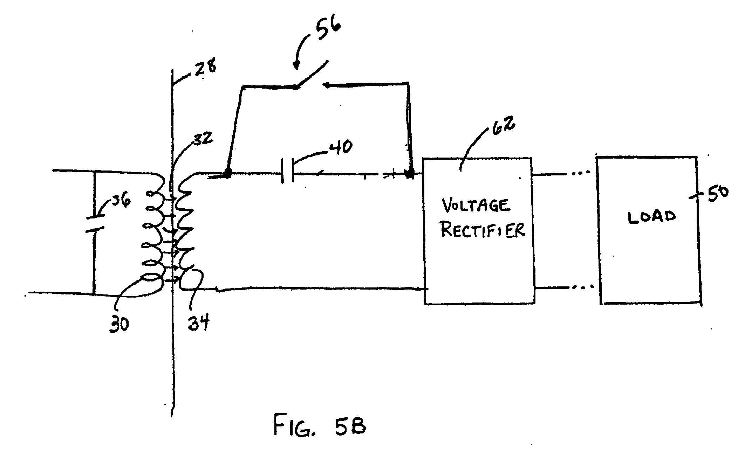

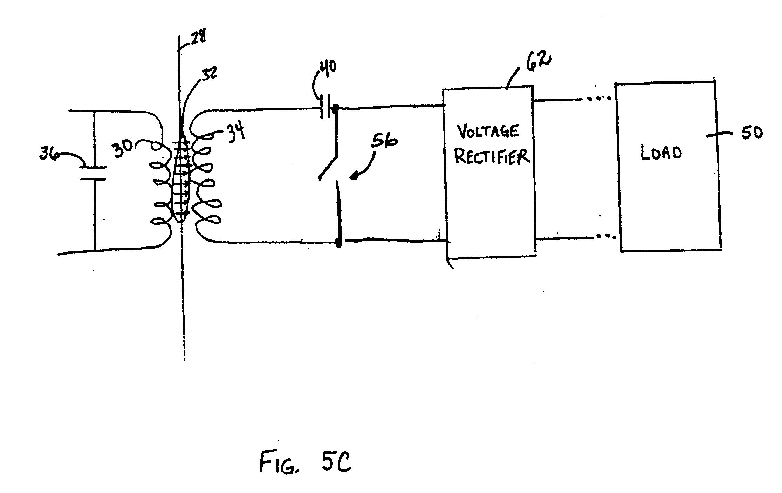

FIG. 5B depicts another exemplary embodiment for selectively detuning secondary resonant circuit 54 when secondary coil 34 and capacitor 40 form a series resonant circuit. In this embodiment, switch 56 is placed in parallel with capacitor 40 to short-circuit the capacitor out of resonant circuit 54 when the switch 56 is turned on. FIG. 5C depicts a third exemplary embodiment for short-circuiting secondary resonant circuit 54 when secondary coil 34 and capacitor 40 are a series resonant circuit. In the FIG. 5C embodiment, switch 56 is placed in series with secondary coil 34 and capacitor 40 to short-circuit resonant circuit 54 and prevent energy transfer from the coil to load 50. Switch 56 is controlled by an output signal from comparator 66 to pulse width modulate the energy transferred from secondary coil 34 to full-wave rectifier 62.

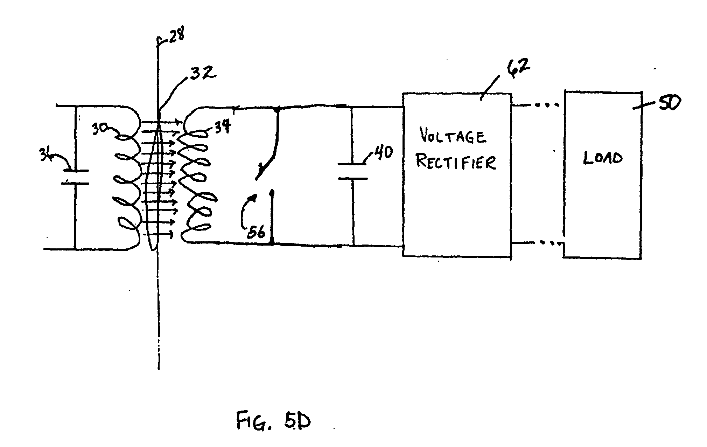

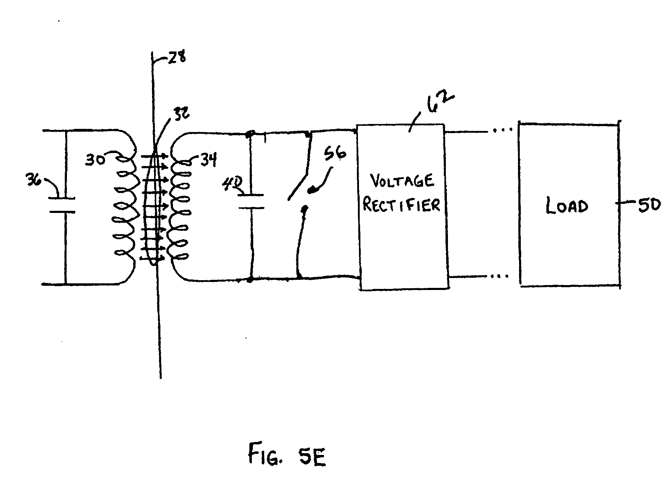

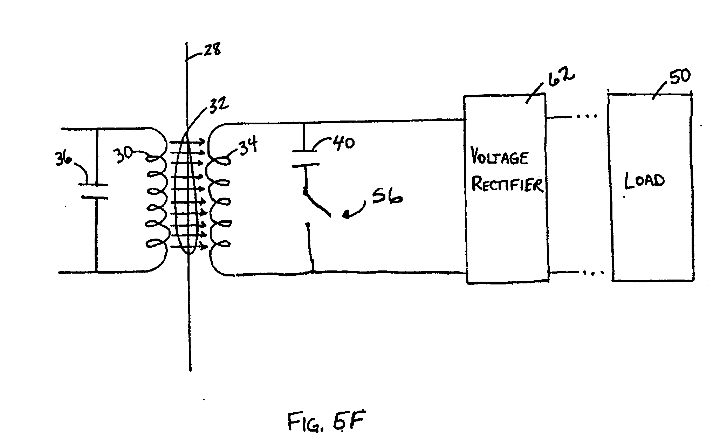

FIGS. 5D-5F depict several embodiments for selectively detuning secondary resonant circuit 54 and, thus, regulating power transfer when secondary coil 34 and capacitor 40 are connected as a parallel resonant circuit. In FIG. 5D, switch 56 is connected in parallel between secondary coil 34 and capacitor 40 to effectively short-circuit capacitor 40 out of the circuit when the switch 56 is turned on. In FIG. 5E, switch 56 is placed in parallel with secondary coil 34 and capacitor 40 between secondary resonant circuit 54 and voltage rectifier 62. This embodiment is similar to that provided in FIG. 5C, in that when turned on, switch 56 short-circuits resonant circuit 54 and prevents energy transfer from secondary coil 34 to load 50. In FIG. 5F, switch 56 is placed in series with capacitor 40 to short-circuit the capacitor from resonant circuit 54 when switch 56 is turned on.

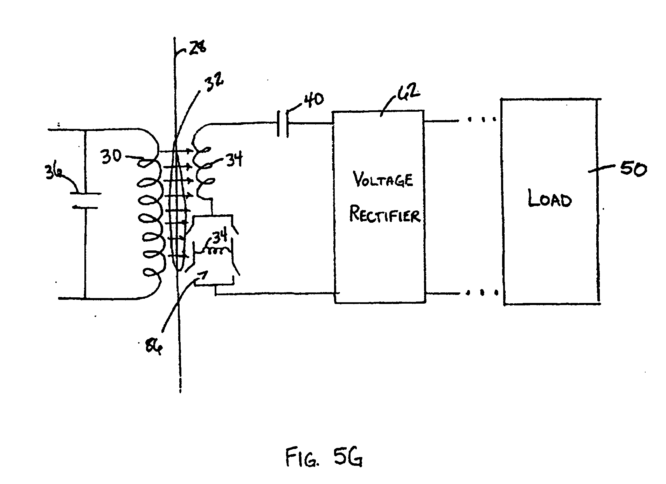

FIG. 5G depicts another exemplary circuit topology for detuning secondary resonant circuit 54 when secondary coil 34 is too large of a load to short circuit using one of the other embodiments described above. In this embodiment, secondary coil 34 is divided into two sections and one section is placed in an H-bridge 86. Pairs of switches in the H-bridge are alternately closed and opened to effectively reverse one-half of secondary coil 34 in and out of the circuit. When the switches are closed, such that one-half of secondary coil 34 is reversed relative to the other half, the two coil halves electrically cancel each other, effectively turning secondary coil 34 off when the transfer power exceeds the threshold voltage.

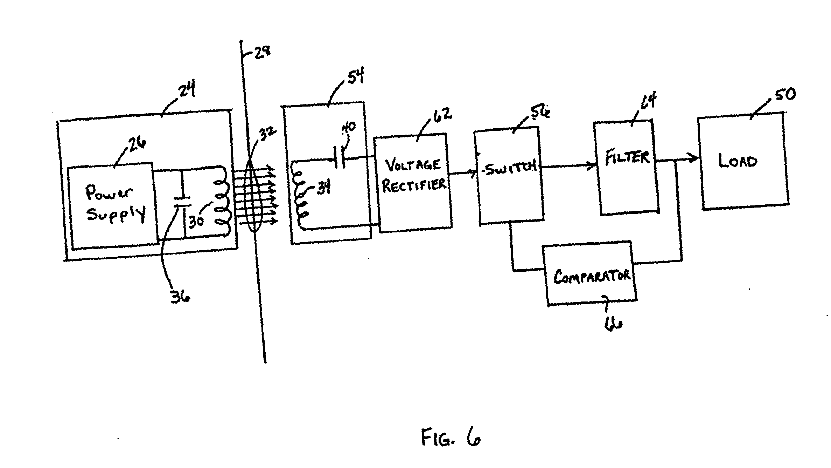

FIG. 6 depicts a second embodiment for the present invention, in which switch 56 is located between full-wave voltage rectifier 62 and filter capacitors 64 to modulate the rectified power signal. In the first embodiment described above, switch 56 short-circuits either secondary coil 34 or capacitor 40 to selectively decouple resonant circuit 54 and thereby regulate the transfer power. In the second embodiment shown in FIG. 6, switch 56 is positioned between voltage rectifier 62 and filter capacitors 64 to pulse width modulate the rectified power signal. When switch 56 is closed, power is drawn from secondary coil 34, rectified, and transferred to load 50 through filter capacitors 64. When switch 56 is opened, the power transfer circuit is open-circuited and power is not drawn from the secondary coil. While switch 56 is opened, filter capacitors 64 discharge and provide power to load 50. After the load voltage drops below the threshold level, switch 56 is closed, and power transfer is resumed. Filter capacitors 64 recharge as power is transferred from coil 34 to load 50.

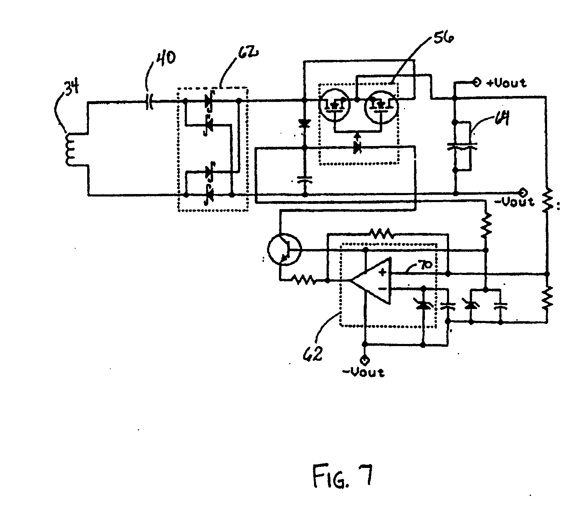

FIG. 7 provides a detailed schematic diagram illustrating the second embodiment of the invention. The schematic in FIG. 7 is similar to the schematic in FIG. 4 except for the relocation of switch 56. As shown in FIG. 7, in this exemplary embodiment switch 56 comprises a solid-state relay between full-wave rectifier 62 and filter capacitors 64. An output signal from comparator 66 turns the relay on and off, based upon the output power to load 50. While switch 56 is depicted as a solid-state relay, numerous other types of switching devices could also be used to accomplish the present invention.

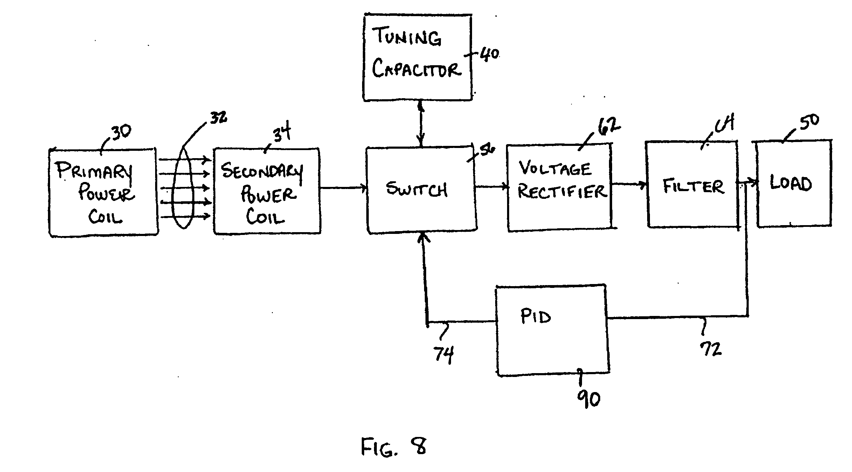

FIG. 8 illustrates an alternative embodiment for the power control circuit 52 of the present invention. In the alternative embodiment, comparator 66 in the closed loop power control system is replaced with a Proportional, Integral, Derivative (PID) controller 90. PID controller 90 activates switch 56 to pulse width modulate the power signal. PID controller 90 modulates the power signal by first calculating the error between the actual voltage in load output signal 72 and voltage threshold 70. This error is multiplied by the proportional gain, then integrated with respect to time and multiplied by the integral gain. Finally, the error is differentiated with respect to time and multiplied by the differential gain of controller 90 to generate a control signal 74 for switch 56. Control signal 74 will continually vary based upon the amplifier gains. Controller 90 operates at a fixed frequency and determines the amount of time to open and close switch 56 during each duty cycle, based upon the gains acting upon the error signal. By operating at fixed frequency intervals, the PID controller 90 responds quickly to changes in the power levels and provides increased control over the pulse width modulation of the power signal.

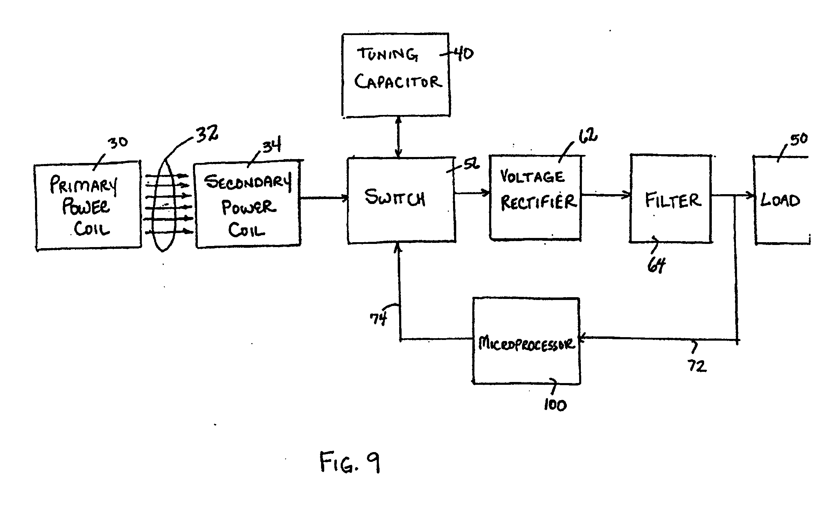

FIG. 9 illustrates another alternative embodiment for the present invention, in which a microcontroller 100 is utilized to control the difference between the voltage of output signal 72 and a desired voltage level. From this difference, microprocessor 100 digitally controls switch 56 to modulate the power signal. Microprocessor 100 provides precision control over the selective detuning of secondary resonant circuit 54 and, thus, a stable load power. While FIGS. 9 and 10 depict switch 56 in the first embodiment position, where the switch selectively detunes resonant circuit 54, PID controller 90 and microprocessor 100 may also be used in the closed loop control of the second embodiment described above, in which switch 56 is positioned between voltage rectifier 62 and filter capacitors 64.

It should be appreciated that various loads 50 of an implant device 22 may benefit from regulating transferred power, to include both maintaining voltage within certain parameters and current within certain parameters. Thus, sensing current may be used as an alternative to, or in addition to, sensing voltage.

While the present invention has been illustrated by description of several embodiments and while the illustrative embodiments have been described in considerable detail, it is not the intention of the applicant to restrict or in any way limit the scope of the appended claims to such detail. Additional advantages and modifications may readily appear to those skilled in the art.

For example, implantable, bi-directional infusing devices that would benefit from enhanced TET powering and telemetry are disclosed in EP 1 600 183 A1, EP 1 600 120 A1, EP 1 600 119 A1, and EP 1 600 125 A1.

## Claims

1. An implantable medical device (22) for receiving a transcutaneous energy transfer (TET) signal from a primary circuit (24) at a resonance frequency, the implantable medical device comprising:
an active load (50) requiring a supply power;
a secondary coil (34) coupled to capacitance (40) selected to form a tuning circuit (54) responsive to the TET signal to produce a received signal;
circuitry coupled to the tuning circuit (54) and operatively configured to convert the received signal into a supply power for the active load (50);
detuning circuitry; and
power control circuitry (52) responsive to a sensed value of the supply power to selectively switch the detuning circuit into electrical communication with the secondary coil (34) to reduce a power transfer characteristic of the received signal, wherein the power control circuitry comprises a voltage comparator (66) **characterized by** the voltage comparator (66) comprising a pulse width modulation controller operably configured to adjust a duty cycle defined by sequential periods when the detuning circuitry is in electrical communication with the secondary coil (34) to reduce the power transfer characteristic.

2. The implantable medical device of claim 1, wherein a secondary coil (34) includes a first and second secondary coil, the detuning circuit comprises a switching circuit (86) operably configured to serially connect the second secondary coil selectively between a first orientation and a second orientation that is electrically reversed from the first orientation.

3. The implantable medical device of claim 1, wherein the detuning circuit comprises a tuning capacitor (40).

4. The implantable medical device of claim 1, wherein the tuning circuit (54) comprises a tuning capacitor (40) series coupled to the secondary coil (34) to form a detuned resonance condition, the medical device further comprising a solid-state relay (56) operatively configured to respond to the voltage comparator (66) by selectively shorting across the tuning capacitor (40) to return the secondary coil (34) to a resonance frequency condition.

5. The implantable medical device of claim 1, wherein the pulse width modulation controller comprises a Proportional Integral Derivative controller (90).

6. The implantable medical device of claim 1 wherein the circuitry operatively configured to convert the received signal comprises a rectifier (62).

7. The implantable medical device of claim 6, wherein the rectifier (62) and detuning circuitry comprise a switch circuit responsive to the voltage comparator (66) to selectively couple a rectified power supply signal to the active load (50) and to short circuit the secondary coil (34).

8. A transcutaneous energy transfer (TET) system (20), comprising:
an external portion, comprising:
a primary circuit (24) operably configured to resonate at a resonance frequency,
an excitation circuit (38) in electrical communication with the primary circuit (24) and operably configured to create an alternating magnetic field at the resonance frequency; and
an implantable medical device (22) of any preceding claims, wherein:
the active load requires a supply power having electrical parameters within respective ranges.

## Patentansprüche

1. Implantierbare medizinische Vorrichtung (22) zum Empfangen eines Signals eines transkutanen Energieübertrags (TET) von einer primären Schaltung (24) bei einer Resonanzfrequenz, wobei die implantierbare medizinische Vorrichtung Folgendes umfasst:
eine aktive Last (50), die eine Energieversorgung benötigt;
eine sekundäre Spule (34), die mit einer Kapazität (40) gekoppelt ist, welche ausgewählt ist, um eine Abstimmschaltung (54) zu bilden, die auf das TET-Signal anspricht, um ein Empfangssignal zu erzeugen;
eine Schaltung, die mit der Abstimmschaltung (54) gekoppelt ist und funktionell dafür eingerichtet ist, das empfangene Signal in eine Energieversorgung für die aktive Last (50) umzuwandeln;
eine Verstimmschaltung; und
eine Energiesteuerschaltung (52), welche auf einem gemessenen Wert der Energieversorgung anspricht, um wahlweise die Verstimmschaltung in elektrische Verbindung mit der sekundären Spule (34) zu schalten, um die Energieübertragungscharakteristik des Empfangssignals zu reduzieren, wobei die Energiesteuerschaltung einen Spannungsvergleicher (66) umfasst, **dadurch gekennzeichnet, dass** der Spannungsvergleicher (66) eine Pulsbreitenmodulationssteuerung umfasst, die funktionell dafür eingerichtet ist, einen Arbeitszyklus einzustellen, der durch sequentielle Perioden definiert ist, wenn die Verstimmschaltung in elektrischer Verbindung mit der sekundären Spule (34) ist, um die Energieübertragungscharakteristik zu reduzieren.

2. Implantierbare medizinische Vorrichtung nach Anspruch 1, wobei die sekundäre Spule (34) eine erste und eine zweite sekundäre Spule aufweist und die Verstimmschaltung eine Umschaltschaltung (86) umfasst, die funktionell dafür eingerichtet ist, die zweite sekundäre Spule wahlweise zwischen einer ersten Orientierung und einer zweiten Orientierung, die elektrisch umgekehrt zu der ersten Orientierung ist, seriell zu verbinden.

3. Implantierbare medizinische Vorrichtung nach Anspruch 1, wobei die Verstimmschaltung einen Abstimmkondensator (40) umfasst.

4. Implantierbare medizinische Vorrichtung nach Anspruch 1, wobei die Abstimmschaltung (54) einen Abstimmkondensator (40) in Serie umfasst, der mit der sekundären Spule (34) gekoppelt ist, um eine verstimmte Resonanzbedingung zu bilden, wobei die medizinische Vorrichtung weiterhin ein Halbleiterrelais (56) aufweist, welches funktionell dafür eingerichtet ist, auf den Spannungsvergleicher (66) durch wahlweises Kurzschließen des Abstimmkondensators (40) zu reagieren, um die sekundäre Spule (34) in eine Resonanzfrequenzbedingung zurück zu bringen.

5. Implantierbare medizinische Vorrichtung nach Anspruch 1, wobei die Pulsbreitenmodulationssteuerung einen Proportional-Integral-Differential-Regler (90) umfasst.

6. Implantierbare medizinische Vorrichtung nach Anspruch 1, wobei die Schaltung, welche funktionell dafür eingerichtet ist, das empfangene Signal umzuwandeln, einen Gleichrichter (62) umfasst.

7. Implantierbare medizinische Vorrichtung nach Anspruch 6, wobei der Gleichrichter (62) und die Verstimmschaltung eine Umschaltschaltung aufweisen, die auf den Spannungsvergleicher (66) reagiert, um wahlweise ein gleichgerichtetes Energieversorgungssignal mit der aktiven Last (50) zu koppeln und die sekundäre Spule (34) kurzzuschließen.

8. System (20) zur transkutanen Energieübertragung (TET), das Folgendes umfasst:
einen externen Teil, der Folgendes umfasst:
eine primäre Schaltung (24), die funktionell dafür eingerichtet ist, bei einer Resonanzfrequenz zu schwingen,
eine Erregerschaltung (38), die in elektrischer Verbindung mit der primären Schaltung (24) steht und funktionell dafür eingerichtet ist, ein magnetisches Wechselfeld bei der Resonanzfrequenz zu erzeugen; und
eine implantierbare medizinische Vorrichtung (22) nach einem der vorhergehenden Ansprüche, wobei
die aktive Last eine Energieversorgung erfordert, die elektrische Parameter innerhalb entsprechender Grenzen aufweist.

## Revendications

1. Dispositif médical implantable (22) pour recevoir un signal de transmission d'énergie par voie transcutané (TET) depuis un circuit primaire (24) à une fréquence de résonance, le dispositif médical implantable comprenant :
- une charge active (50) nécessitant une source d'énergie ;
- une bobine secondaire (34) couplée à une capacité (40) choisie pour former un circuit d'accord (54) répondant au signal de TET pour produire un signal reçu ;
- un système de circuits couplé au circuit d'accord (54) et configuré fonctionnellement pour convertir le signal reçu en une source d'énergie pour la charge active (50) ;
- un système de circuits de désaccord ; et
- un système de circuits de commande de l'énergie (52) répondant à une valeur captée de la source d'énergie pour commuter sélectivement le circuit de désaccord en une communication électrique que la bobine secondaire (34) pour réduire une caractéristique de transmission de puissance du signal reçu, le système de circuits de commande de l'énergie comprenant un comparateur de tensions (66), **caractérisé en ce que** le comparateur de tensions (66) comprend un dispositif de contrôle de la modulation de la largeur d'impulsion configuré fonctionnellement de façon à ajuster un cycle de service défini par des périodes séquentielles lorsque le système de circuits de désaccord est en communication électrique avec la bobine secondaire (34) pour réduire la caractéristique de transmission d'énergie.

2. Dispositif médical implantable selon la revendication 1, dans lequel une bobine secondaire (34) comprend une première et une deuxième bobine secondaire, le circuit de désaccord comprend un circuit de commutation (86) configuré fonctionnellement pour relier en série la deuxième bobine secondaire sélectivement entre une première orientation et une deuxième orientation qui est électriquement inversée de la première orientation.

3. Dispositif médical implantable selon la revendication 1, dans lequel le circuit de désaccord comprend un condensateur d'accord (40).

4. Dispositif médical implantable selon la revendication 1, dans lequel le circuit d'accord (54) comprend un condensateur d'accord (40) couplé en série à la deuxième bobine (34) pour former un état de résonance désaccordé, le dispositif médical comprenant en outre un relais à l'état solide (56) configuré fonctionnellement pour répondre au comparateur de tension (66) par raccourcissement sélectif sur le condensateur d'accord (40) pour faire repasser la bobine secondaire (34) à un état de fréquence de résonance.

5. Dispositif médical implantable selon la revendication 1, dans lequel le dispositif de contrôle de la modulation de la largeur d'impulsion comprend un dispositif de contrôle du proportionnel intégral dérivé (90).

6. Dispositif médical implantable selon la revendication 1, dans lequel le système de circuits configuré fonctionnellement de façon à convertir le signal reçu comprend un rectificateur (62).

7. Dispositif médical implantable selon la revendication 6, dans lequel le rectificateur (62) et le système de circuits de désaccord comprennent un circuit de commutation répondant au comparateur de tension (66) pour coupler sélectivement une source de signal de la source d'énergie rectifié à la charge active (50) et pour court-circuiter la bobine secondaire (34).

8. Système de transmission d'énergie par voie transcutanée (TET) (20) comprenant :
une partie externe, comprenant :
- un circuit primaire (24) configuré fonctionnellement pour résonner à une fréquence de résonance,
- un circuit d'excitation (38) en communication électrique avec le circuit primaire (24) et configuré fonctionnellement pour créer un champ magnétique alternatif à la fréquence de résonance ; et
- un dispositif médical implantable (22) selon l'une quelconque des revendications précédentes, dans lequel :
- la charge active nécessite une source d'énergie ayant des paramètres électriques dans des ordres de grandeur respectifs.
